# EUROPEAN PATENT APPLICATION

(11) **EP 4 710 843 A1**
(43) Date of publication of application: **18.03.2026**
(21) Application number: 24200933.0
(22) Date of filing: 17.09.2024
(51) Int. Cl.: A61B 5/00, A61B 3/10

(54) **METHOD AND SYSTEM FOR PROVIDING FEEDBACK TO A USER USING AN OCT IMAGING SYSTEM**

(71) Applicant: Carl Zeiss Meditec AG, 07745 Jena (DE)
(72) Inventor: MacDougall, Daniel, Halifax, B3H0A8 (CA); Adamson, Robert, Halifax, B3H0A8 (CA); Hubley, Drew, Halifax, B3H0A8 (CA); Tingley, Becca, Halifax, B3H0A8 (CA); Farrell, Josh, Halifax, B3H0A8 (CA)
(74) Representative: Carl Zeiss AG - Patentabteilung

(57) **Abstract**

Disclosed herein are system, apparatus, article of manufacture, and/or method embodiments, and/or combinations and sub-combinations thereof, for a computer-implemented method for providing feedback to a user via an OCT imaging system (120, 122) including generating a mechanical stimulus to induce vibrations on an anatomical structure (210) in a body (130), receiving OCT signal data from a measured location (230) in the body, wherein the OCT signal data comprises a vibrometry measurement of a vibration of the anatomical structure at the measured location (230), determining a displacement (240), wherein the displacement is a spatial deviation by which a target location (220) is displaced from the measured location (230), and providing feedback to the user via a user interface (140, 430, 608, 382, 384), wherein the feedback comprises visualization (140, 440) of the displacement (240).

## Description

### TECHNICAL FIELD

The present disclosure is generally directed to providing feedback to a user making measurements using an optical coherence tomography (OCT) imaging system.

### SUMMARY

Otoscopy is a well-known clinical procedure used to examine structures of the ear. However, otoscopy has limitations such as poor visualization or inadequate time for evaluation in suboptimal environments. Recently, new devices have been developed to improve early and reliable diagnosis of various ear diseases. Spatially resolved and three-dimensional measurements of structures within the ear are desirable. Newer technologies may be used for performing diagnostic procedures using OCT technology for subsurface imaging of the ear.

OCT imaging of the inner ear may be performed with certain handheld devices. Making measurements while a tip of a handpiece as part of an optical imaging system is in the ear canal may have certain limitations or inefficiencies as a human user performs the measurement. For example, during measurements of structures of the inner ear, the user's hand may move slightly. These movements may cause the handpiece and thus the measurement to not be on track of a desired location to be measured or create images of insufficient quality. Users with little experience of conducting measurements and/or with limited knowledge of the anatomy of the inner ear might face problems in making a measurement of the desired anatomical structure and/or in finding a correct measurement location quickly or reliably. This can happen even though state-of-the-art systems comprise live view / real-time imaging capabilities.

It is therefore an object of the present disclosure to provide an improved method and/or device for providing feedback to a user making measurements of anatomical structures in a body using an OCT imaging system. This object is achieved by a computer-implemented method for providing feedback to a user according to Claim 1, a computer program product for providing feedback to a user according to Claim 14 and a device for providing feedback to a user according to Claim 15.

Provided herein are system, apparatus, and/or method embodiments, and/or combinations and sub-combinations thereof, for providing feedback to a user making measurements using an OCT imaging system.

According to an aspect of the present disclosure, a computer-implemented method for providing feedback to a user via an OCT imaging system includes generating a mechanical stimulus to induce vibrations on an anatomical structure in a body, receiving OCT signal data from a measured location in the body, wherein the OCT signal data comprise a vibrometry measurement of a vibration of the anatomical structure at the measured location, determining a displacement from the OCT signal data, wherein the displacement is a spatial deviation by which the measured location in the body is displaced from a target location in the body and providing feedback to the user via a user interface, wherein the feedback comprises visualization of the displacement.

The "mechanical stimulus" is an impulse created by mechanical energy. It can, for example, be based on sound pressure (acoustic stimulus) or on air pressure. For example, sound waves or a volume of pressurized air can be emitted by a mechanical stimulus source such as a speaker or an air pressure generator in a direction towards the anatomical structure which cause the structure to vibrate in response to the stimulus. Excitating individual ossicles of the inner ear as anatomical structures to vibrate and measuring them during vibration using an OCT imaging device is a known technique to assess a functioning of ossicles and detect potential pathologies. It is applied in combined OCT and Doppler vibrometry systems.

The OCT signal data can be a line scan ("A scan") of the measured location which is acquired by the OCT imaging system. Alternatively, the OCT signal data can be multiple line scans, e. g. a full "B scan" of a tissue. The imaging system based on OCT technology can image the vibrating (part of) structure if (a) the field of view of the imaging system, e. g. determined by a handpiece with an associated screen, is oriented such that it captures the structure and if (b) the depth of focus is configured to depict the structure in a volume of the patient's body.

The "spatial deviation" may include a distance and/or an orientation, e. g. a solid angle, by which a target location within a patient's body that is intended to be measured is displaced from a location that has been (erroneously) measured in the patient's body. The "displacement" or "spatial deviation" may occur before or during an acquisition of OCT signal data from the body. A double displacement before and during a measurement is also possible. If the displacement occurs before and not during a measurement, the measured location may be imaged in sufficient quality. However, the "spatial deviation" leads to a situation in which the measured location is not identical to the target location that is intended to be measured. This can mean that an ossicle in an inner ear of the patient as a target location is not fully depicted or not depicted at all or depicted in a wrong plane in an OCT image. In contrast to that, if the displacement occurs during a measurement, the spatial deviation can lead to an insufficient image quality if a distance and/or speed of movement is too high in relationship to a frequency of acquiring OCT signal data (e. g. A-scans which are stitched together to form a B-scan). In other words, an OCT image created in this situation includes image information of more than the target location in the patient's body. In this case, the measured location may include a target location and additionally locations in the vicinity of the target location. It depends on the movement and on the OCT imaging frequency to which extent the image quality is affected negatively and how much the measured location, or boundaries of the measured location deviate(s) from the target location.

The feedback to the user can, in a basic implementation, include a piece of information that a displacement is present or has been detected. Preferably, it includes a calculated or estimated quantification of the displacement, i. e. a distance value and/or a solid angle value. Receiving this enriched information enables the user to correct a displacement in a subsequent measurement more easily compared to a basic information that a displacement has happened. Preferably, for determining a quantification, at least one point of reference at the anatomical structure is determined. Further, it can be advantageous to use a predefined measurement method in order to quantify the displacement in a repeatable manner. According to an even more preferred embodiment of the disclosure, the feedback includes a recommendation to the user of how to minimize or avoid a displacement in a controlled manner before and/or during conducting a subsequent measurement step. For example, a direction, a distance, and/or an angle in which a handpiece should be moved to make a correct measurement can be displayed. When reaching a correct position of measuring the target location, a notification might be output to the user. Correct or incorrect positioning might be indicated by using vibrational or acoustic notifications in addition to visual notifications. Feedback to the user can be issued stepwise, e. g. after each measurement. Alternatively, the feedback is given live / online, i. e. during a positioning procedure of a handpiece with a corresponding field of view of the OCT imaging functionality. Preferably, live and post-measurement feedback are combined to ease the process of finding a correct measurement location for the user and to secure that a measurement cycle of a series of single measurements has been conducted according to predefined quality requirements.

The user interface can be a display which is an integral or connected part of the OCT imaging system. For example, it can be an external display/screen or a display integrated in a handpiece.

The present disclosure is advantageous because a visual feedback to the user on a displacement is a reliable and easily understandable means of indicating an error. A visualization can help the user to correct an error in a next measurement step.

According to an aspect of the present disclosure, a computer program product for providing feedback to the user via the OCT imaging system includes a computer-readable storage medium having program instructions provided thereon, the program instructions being executable by one or more computer systems or controllers to cause the one or more computer systems to perform the method.

According to an aspect of the present disclosure, a device for providing feedback to a user via an OCT imaging system includes a mechanical stimulus source configured to generate a mechanical stimulus to induce vibrations on an anatomical structure in a body, an OCT imaging device configured to measure OCT signal data from a measured location in the body, wherein the OCT signal data includes a vibrometry measurement of a vibration of the anatomical structure at the measured location and further includes a processor and a memory connected to the mechanical stimulus source and the OCT imaging system, wherein the processor is configured to execute instructions stored in said memory for performing the steps of determining a displacement from the OCT signal data, wherein the displacement is a spatial deviation by which the measured location in the body is displaced from a target location in the body and of providing feedback to the user via a user interface, wherein the feedback includes visualization of the displacement.

Further particularly advantageous embodiments and further developments of the present disclosure are obtained from the dependent claims and the following description, wherein the independent claims of one claim category can also be further developed similarly to the dependent claims and exemplary embodiments of another claim category and in particular, individual features of various exemplary embodiments or variants can be combined to form new exemplary embodiments or variants.

According to a preferred embodiment of the disclosure, the OCT signal data may include motion artifacts. Motion artifacts occur in images, if an exposure time per frame is too long or a frequency of taking images is too low for depicting a structure in one single state. Motion artifacts can lead to an insufficient image quality. The OCT imaging system may include a software-based method to detect images of insufficient quality and automatically discard them. The presence of motion artifacts is an indicator that at least a displacement or spatial deviation occurred during an acquisition of OCT signal data. It cannot serve as an indicator for whether a displacement or spatial deviation occurred before an acquisition of OCT signal data.

According to a preferred embodiment of the disclosure, the method further includes that a first motion causing the motion artifacts has a lower frequency than a second motion of the vibrating anatomical structure. The first motion can be a tremor or a more irregular movement of a hand holding a handpiece of an OCT imaging system. Alternatively, or additionally, it can be a heartbeat or a breathing movement of the user and/or the patient indirectly moving the hand and/or head. Also, other natural movements of body parts may be included in the first motion. The second motion which is induced by a mechanical stimulus source is of higher frequency compared to the first motion. For example, it can have a value between 100 Hz and 10.000 Hz. The first motion preferably has a frequency of 50 Hz at maximum, more preferably of 20 Hz at maximum. The second motion preferably has a frequency of 60 Hz at minimum, more preferably of 100 Hz at minimum. This embodiment provides the advantage of being able to differentiate natural from voluntarily induced movements of body parts.

According to a preferred embodiment of the disclosure, the method further includes that the motion artifacts relate to a movement of the body relative to an optical path of the OCT imaging system. Potentially, there are two sources of natural movements of body parts, the user and the patient. Movements of those two and different movements within each body can occur at the same time. They can sometimes hardly be isolated from each other but can superimpose on each other or compensate each other at least partly. An acquisition of OCT signal data is affected by the combined movements during a measurement. This can be expressed by a relative movement between the patient's body and the optical path of the OCT imaging system whose handpiece is usually held by a user when taking a measurement. Particularly, this refers to a segment of the optical OCT path extending from a distal end of a tip of the handpiece to a first movable mirror, which segment can have a fixed position within the handpiece.

According to a preferred embodiment of the disclosure, the method further includes that the target location includes at least a portion of the anatomical structure, and the measured location is different from the anatomical structure. The ear ossicles as anatomical structures in the body are excitated to vibrate in order to measure whether they work correctly or not. Therefore, a target location that is intended to be measured is at least a part of the anatomical structure which is made to vibrate. The size of the part or portion of the anatomical structure can depend on whether an A-scan, a B-scan or a volumetric OCT image is taken from the anatomical structure. In some cases, it can be useful to fully depict the anatomical structure. Then the target location includes the anatomical structure in its whole extension, e. g. an incus or malleus. If the measured location is different from anatomical structure, the image of it at least does not include a significant part of it which is characteristic for it. This means that it cannot be recognized from the corresponding image without significant doubt. In this case, an evaluation software of the OCT imaging system may conclude that the OCT signal data have been acquired at a wrong position and that the measurement should be repeated after correcting the position to enable imaging of a target location. For this purpose, the OCT imaging system may include a computer vision or other Artificial Intelligence-based system configured for image feature analysis in OCT images. It may include a previously created software-based library of anatomical structures of the respective body region, e. g. the inner ear, in different planes and/or perspectives. A current measurement or OCT image can be compared to the library and an image matching can be assessed determining an individual accuracy level. If in a comparison of a current image and an image stored in the library a similarity level between these images is not sufficient despite sufficient image sharpness, an image analysis software might conclude and output to a user that a displacement happened and the procedure of taking the image should be repeated.

According to a preferred embodiment of the disclosure, the method further includes that an axial direction and a lateral direction of the displacement relative to a direction of an optical path of the OCT imaging system are determined independently of each other. "Axial direction" means that a displacement in a direction coaxial to the measurement direction of the OCT imaging system can be determined. For example, if a tip of a handpiece points towards the tympanic membrane of a patient's ear and an anatomical structure behind the tympanic membrane is intended to be measured but is not present in an acquired image, this error may be caused by a wrong depth of focus of the OCT. In contrast to that, "lateral direction" means a direction in a plane which is preferably perpendicular to the "axial direction". This embodiment provides the advantage to enable an easier correction of the position for a subsequent measurement. Navigating in a three-dimensional space is a complex task for a user and an independent determination creates the possibility for a specific indication to the user within the context of feedback of how to adjust a position of the handpiece of the OCT imaging system in an axial direction and a lateral direction separate from each other.

According to a preferred embodiment of the disclosure, the method further includes that a displacement in an axial direction of the displacement relative to a direction of an optical path of the OCT imaging system is determined based on coregistration of the OCT signal data and image data generated by the image system. This embodiment provides the advantage that additional data does not need to be acquired to determine a displacement in the axial direction.

According to a preferred embodiment of the disclosure, the method further includes that a displacement in a lateral direction of the displacement relative to a direction of an optical path of the OCT imaging system is based on coregistration of a real-time image and the OCT signal data. The real-time image can depict a target region of the body behind which target region in a direction along (coaxial or parallel) an optical path of the OCT imaging system and towards the interior of the patient's head the anatomical structure is located which includes the target location. The target region can, for example, be a tympanic membrane of a patient's ear and adjacent tissue that can be imaged without using OCT imaging technology and its capability of visualizing subsurface structures. In other words, the real-time images include an anatomical topography (or body surface) which can be seen from a position external to a body volume. For that purpose, an otoscope including an OCT imaging system may include a camera providing images from an ear canal based on real-time imaging. This embodiment eases navigation for the user because healthcare professionals know which anatomical structures are located behind those anatomical structures that are shown in a real-time image of the ear. By coregistration of images from the OCT imaging device and the real-time light imaging device navigation is further facilitated.

According to a preferred embodiment of the disclosure, the method further includes that the feedback to the user includes a recommendation on how to reduce or avoid the displacement, preferably based on an analysis of root causes of the determined displacement. If a software-based evaluation system of the OCT imaging system determines that a root cause is excessive movement of a body part, then a notification to the user may include a caution message to reduce body movements of the user and/or the patients. If the system determines that a root cause is that a wrong location different from the target location has been measured, then a notification to the user may include a caution message to position the otoscope more accurately and preferably adds quantitative information and/or visual assistance which can, for example, include guiding arrows and/or colored signs indicating correct or wrong position changes during positioning of the user.

According to a preferred embodiment of the disclosure, the method further includes generating a real-time image of the anatomical structure and providing the feedback as an overlay to the real-time image. The information overlaid on an image of the OCT-based measurement can include text and/or graphical information. For example, it may include signs, points or lines of reference in the anatomical structure that is intended to be measured and the portion of tissue that has erroneously been measured. The overlay mode which is displayed in a specific situation can be selected from a predefined set of overlays which is displayed depending on a calculated displacement. The overlay can alternatively be generated dynamically depending on a set of criteria. Preferably, the overlaid information corresponds, e. g. is dimensionally adjusted to a live image of the structure. For example, lines or arrows which indicate the displacement are placed and sized in a manner that enables a user to minimize or avoid a displacement in a subsequent measurement cycle more reliably.

According to a preferred embodiment of the disclosure, the method further includes that the determined displacement is communicated to the user if it exceeds a predefined threshold. The predefined threshold can be determined based on a set of intensity values of reflected light from the structure. The set of intensity values may include an intensity distribution across the measured field of view. It may alternatively include maximal and/or minimal intensity values. According to a simple example, a set of intensity values may include one single value, e. g. an average intensity value across an image frame. The predefined threshold is determined and set previous to a measurement of a patient. It is usually not determined during a measurement. Optionally, the threshold can be configured by a user. The phrase "displacement exceeding a predefined threshold" can mean that an anatomy which is intended to be measured cannot be identified sufficiently or does not differentiate sufficiently from other, e. g. adjacent, anatomical structures within the patient's body so that a measurement cannot be attributed to the structure without doubt or a portion of the structure crucial for a diagnosis of an illness does not become sufficiently visible.

According to a preferred embodiment of the disclosure, the method further includes that the anatomical structure is an ossicle of a middle ear.

Provided herein are system, apparatus, and/or method embodiments, and/or combinations and sub-combinations thereof, for providing feedback to a user making measurements using an optical coherence tomography (OCT) imaging system or a combined OCT imaging and vibrometry diagnostic system.

OCT is an optical interferometric imaging technology that may produce depth-resolved images of sub-surface tissue structures, such as structures within the ear. This may be accomplished by taking a broadband light-source and splitting it between a reference beam and a sample probing beam. Light that is backscattered from the structures within the sample is collected and interfered (combined) with the reference beam light in order to produce an interference pattern that, once processed, reveals the location of light-reflecting structures in the sample.

OCT has been applied to imaging the ear of patients. Anatomical structures within the ear may be imaged using OCT and functional imaging in the ear by measuring the vibration of middle ear structures in response to sound. Typically, the process includes the estimation of vibration magnitude and/or phase from phase shifts imparted to the OCT light by movement of the anatomical structures of the ear; the acoustic frequency phase variations are then collected over many consecutive complete acoustic cycles and may be analyzed using Fourier analysis. The phase variations determined via the OCT vibrometry system may then be displayed to a user, such as a doctor or nurse.

Such display options of phase variations may include a graphical user interface (GUI) on a display device, such as a computer. The GUI may display measurements, images collected, information regarding the measurements, and provide feedback to the user regarding the measurements. By utilizing the GUI, all information may be available to the user at the same time in the same location. The user may then be able to determine or improve the quality of the measurements they are taking, based on direct feedback via a display including the GUI. For example, once measurements are displayed to the user, the user may be able to determine issues or diseases of the ear based on the measurements. The user may also determine whether or not a new measurement may be required based on various factors received via feedback from the GUI.

In normal operation, patients and users exhibit small, natural movements of the hand and head during measurements. Such movements can affect the operator's ability to target landmarks of interest over the course of measurements. Since such imperfect targeting negatively affects the accuracy of vibrometry measurements, patient heartbeat and breathing and operator steadiness can require that poor quality measurements be discarded and repeated. Additionally, users may wish to discard any measurements that were erroneously taken at locations other than the region of interest, for example, if the wrong landmark was targeted. A new measurement may be required due to unintentional measuring in a location other than a region of interest.

### BRIEF DESCRIPTION OF THE FIGURES

The accompanying drawings are incorporated herein and form a part of the specification.
FIG. 1 illustrates a system for providing feedback to a user while making measurements, according to some embodiments.
FIG. 2 illustrates a system for measuring a displacement of a measurement at a location to a measured location, according to some embodiments.
FIG. 3 illustrates a GUI displaying a conductive hearing loss assessment, according to some embodiments.
FIG. 4 illustrates a flowchart for providing feedback to a user making measurements using an OCT vibrometry system, according to some embodiments.
FIG. 5 illustrates a GUI displaying feedback to a user, according to some embodiments.
FIG.6 illustrates a method for providing feedback to a user making measurements using an OCT vibrometry system, according to some embodiments.
FIG. 7 illustrates an example computer system useful for implementing various embodiments, according to some embodiments.

In the drawings, like reference numbers generally indicate identical or similar elements. Additionally, generally, the left-most digit(s) of a reference number identifies the drawing in which the reference number first appears.

### DETAILED DESCRIPTION

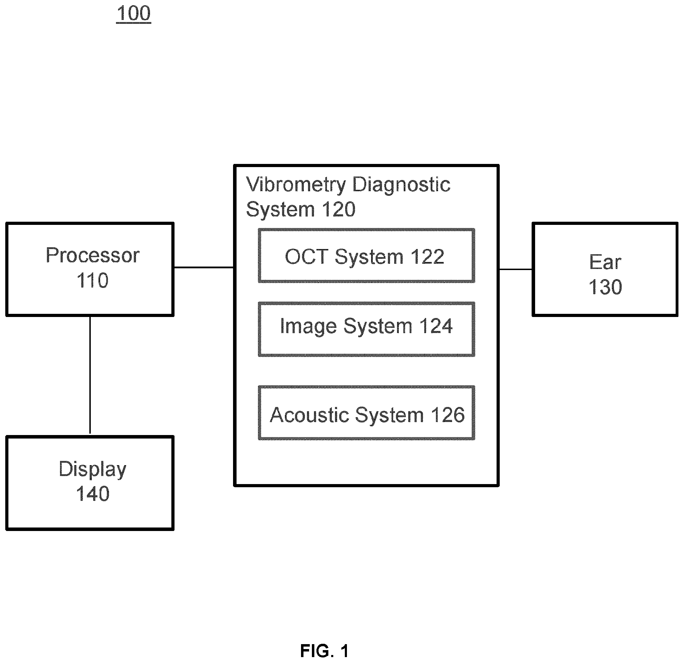

FIG. 1 illustrates a system 100 for providing feedback to a user while making measurements, according to some embodiments. System 100 includes a processor 110 connected to a display 140 and a vibrometry diagnostic system 120 including an OCT system 122, an image system 124 configured to generate real-time images, and an acoustic system 126 to be used measuring a structure within an ear 130.

A vibrometry diagnostic system 120 may be used by a user, such as a medical professional, a doctor, or a nurse, may be handheld, and be inserted into the ear 130 of a patient to perform various measurements on tissues within the ear 130. The vibrometry diagnostic system 120 may include, but is not limited to, an OCT system 122, an image system 124, and an acoustic system 126. The acoustic system 126 may generate an acoustic stimulus at a plurality of frequencies within the ear 130. The frequencies of the acoustic stimulus may range between 125 and 8000 Hz. The acoustic system 126 may be used to measure the sound pressure levels, via a microphone, of the frequencies near structures within the ear 130.

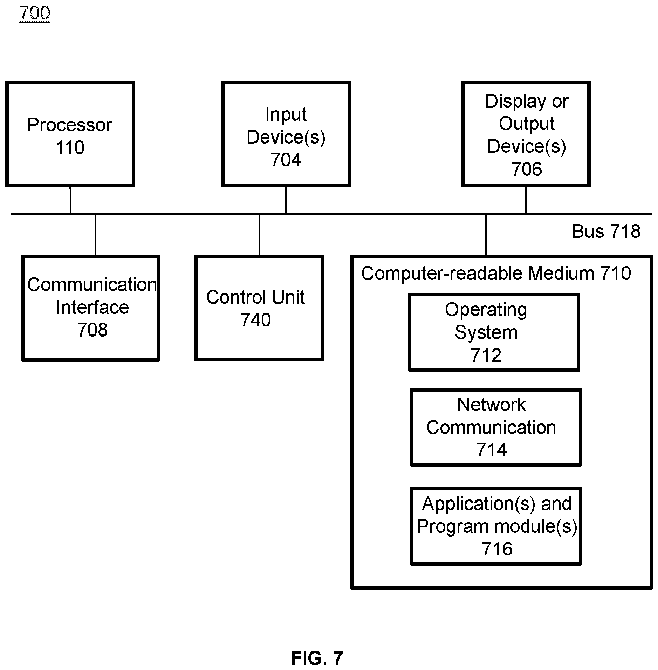

The OCT system 122 may include a light source and an OCT sensor system, an interferometer configured to generate a sample beam and reference beam, and a detector to detect an interfered reference beam and scattered light, a scanning system for scanning the sample beam on the region of interest, such as the ear 130. Based on the acoustic stimulus generated, the OCT system 122 may receive signals and generate images and displayed to the user on a display 140 via the processor 110. The processor 110 is described in further detail in FIG. 7.

When using the OCT system 122 to generate images, the images may correspond to a B-mode image of the ear 130 and may include lines to indicate the depth selected for imaging. The B-mode image may be based on the acoustic stimulus generated from the acoustic system 126, a plurality of A-scans from the OCT system 122, and a plurality of sample interferograms.

Based on the frequency used during a measurement of the ear 130, a mobility measurement may be determined, via the processor 110. The mobility measurement may be based on an amplitude of the vibration induced on the structure from the acoustic system 126 and sound pressure driving the vibration of the structure in the ear 130 at the frequency. The mobility measurement may be measured multiple times at multiple frequencies thereby generating a plurality of mobility measurements and displayed the user on the display 140 via the processor 110. The mobility measurement can be described in terms of acceleration with regards to frequency response.

In some embodiments, the processor 110 may generate a plot of the plurality of mobility measurements and the plot may be displayed to the user on the display 140. The plot may be updated when new frequencies are measured, and new mobility measurements are calculated based on the new frequencies.

The mobility measurement may be calculated at multiple structures within the ear 130, such as the umbo, malleus, the incus, or the stapes. These measurements may also be calculated for the left or the right ear 130 separately. Typically, at least one measurement, preferably up to four measurements, preferably more, should be collected at each region of interest within the ear 130 at a particular frequency. For example, for the left umbo of the ear 130, a measurement at 250Hz, 500 Hz, 1000 Hz, and 2000 Hz may be collected. Since four measurements are collected at each frequency, a total of at least 16 measurements may be collected. However, based on the patient, more or less measurements may be taken or measurements at other structures may be taken.

The number of measurements taken at different frequencies may be displayed to the user on the display 140. Information may be displayed on the display 140 including further details about the measurements taken, but not limited to, what part of the ear 130 is being measured, what frequency is the measurement occurring at, what measurement number is being taken, etc.

An image system 124 may be used simultaneously with the OCT system 122. The image system 124 may include an image sensor and a light source. The image system 124 may be a charged coupled device (CCD), video, thermal, or infrared image and may capture and record real-time images of structures or tissues within the ear 130. The image system 124 may share a common beam path with the OCT system 122 such that the same region of interest is imaged within the ear 130.

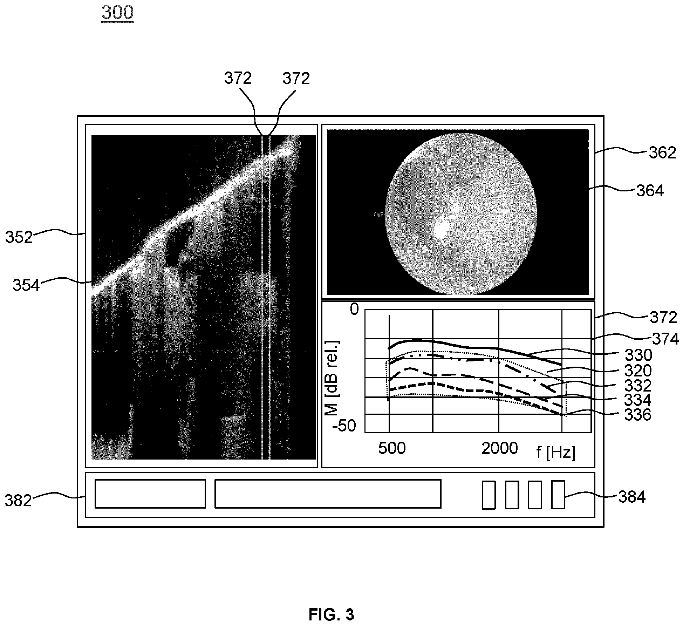

The image system 124 may generate real-time images and may be displayed in the display 140 via the processor 110. The images may display the tympanic membrane, malleus, incus, stapes, or umbo of the patient and is depicted in FIG. 3. While the embodiments described herein are related to the ear, measuring across other tissues in the human body may be envisioned.

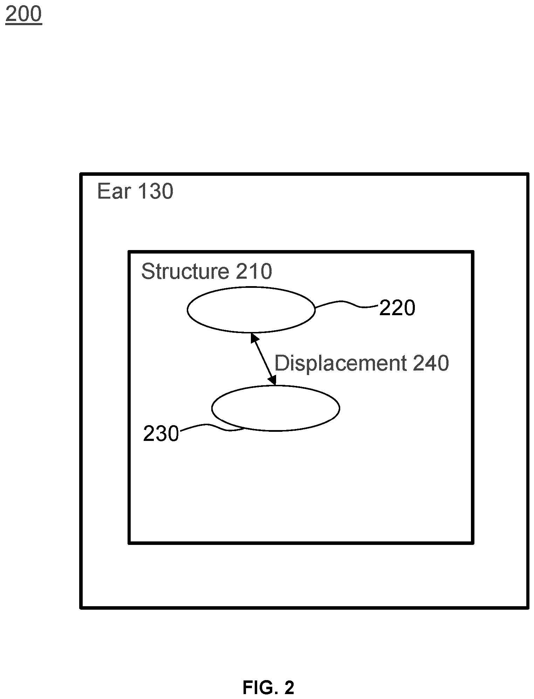

FIG. 2 illustrates a system 200 for measuring a displacement of a measurement at a location to a measured location, according to some embodiments. System 200 includes an ear 130 with a structure 210, a location 220 (also named target location), a measured location 230, and a displacement 240.

The user may perform a measurement on the ear 130 of a patient as described herein. The user may want to measure a structure 210 within the ear 130. The structure 210 may be, for example, umbo, malleus, the incus, or the stapes. The progress of the measurement may be indicated in the display 140 and further described in FIG. 3. In some embodiments, an acoustic stimulus may be generated from the acoustic system 126. The OCT system 122 may receive at least one signal from a measured location 230 on the structure 210 based on the acoustic stimulus generated. More than one signal may be received from the measured location 230 and the signal may include a vibrometry measurement of a vibration induced at the structure 210.

When the measurement is complete, a plot may be generated, displayed in display 140 and further described in FIG. 3. Based on the measurement at the measured location 230, the processor 110 may calculate a displacement 240. The displacement 240 may be a distance and/or angle by which the measured location 230 is displaced from a location 220. The location 220 may be an actual region of interest of the structure 210, i. e. a target location.

The displacement 240 may be relative to a starting location, such as location 220 versus the measured location 230. The user will want to minimize the offsets between the location 220 and the measured location 230.

The displacement 240 may be calculated via an algorithm, a deep learning algorithm, a smoothing filtering algorithm, various mathematical calculations, including known correction calculations such as, without limitation, Kalman filter, image processing algorithms, such Lucas Kanade Sparse Optical Flow algorithm, but not limited to those described herein. The displacement 240 may also be calculated using a Euclidean equation and then averaged out to calculate the global displacement. If this global displacement average is above a set threshold, then feedback may be provided to the user. Other algorithms may include optical flow, multi-frame cross-correlation, or image segmentation and registration.

The displacement 240 may be due to a variety of factors including, but not limited to, the patient moving their head, the patient breathing too heavily or normally, the user moving during the measurement, or measuring a different region on the structure 210. The displacement 240 may be in different planes of the structure 210. The displacement 240 may be in either the axial direction or the lateral direction along the structure 210. Due to the use of OCT system 122, the axial and lateral properties may be decoupled from each other. Specifically, the axial properties may be defined by the coherence properties of the light source and the sampling of the signal at the detector. Lateral properties may be determined from optical properties of the camera system. One depth profile of the sample may be recorded once the reference arm is scanned may be referred to as the A-scan. This may be repeated for each lateral scan.

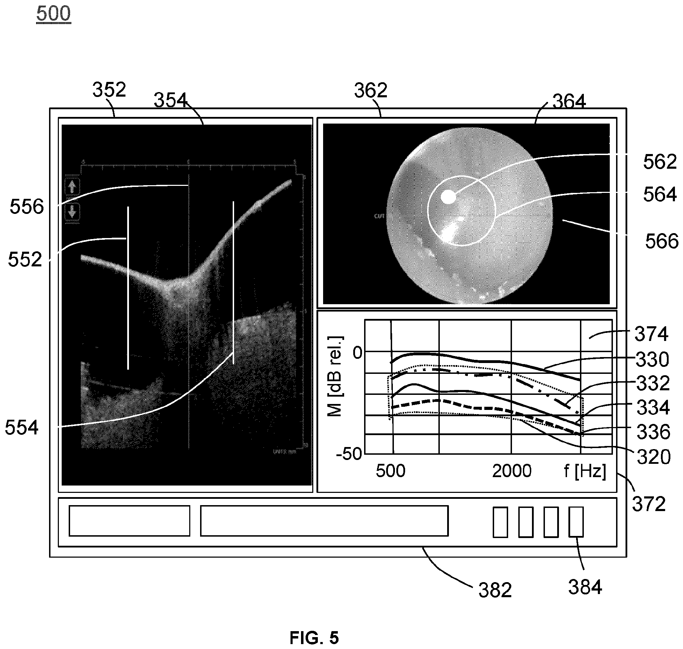

Image information in the axial direction along the A-scan may be reconstructed from an interferometric measurement of delays of light, which may be back-scattered or reflected the ear 130. That is, moving toward or away from the handpiece may be quantified from depth estimates extracted from A scans collected by the OCT system 122 during the measurement. The properties of the light source and the sampling of the interferometric signal may define the axial properties of the OCT system 122. In the axial direction, the displacement 240 may displaced from the axis at which the structure 210 lies. Additionally, the displacement 240 in the axial direction may be based on the coregistration between the images generated by the OCT system 122 and the image system 124 and the images may be aligned or matched from different frequencies to a common reference system. This coregistration, which is further discussed in FIG. 5, ensures that images taken at different times, from different angles, or using different imaging modalities may be aligned such that structure 210 is matched across all images taken. Specifically, the change in parallax from the image system 124 can determine whether the measurement has moved away in the axial direction.

In the lateral direction, the image system 124 can be used to capture a multitude of frames of an en face view of the patient. By capturing a multitude of frames through the duration of a measurement sequence and quantifying the relative movement between those frames using algorithms as described herein, the lateral drift over the course of the measurement period may be quantified and displayed. The movement may be visualized by a numerical quantity (such as maximum deviation, rms (root mean square) deviation, a percentage deviation, or the like) or by a physical path drawn as an overlay directly representing the movement path over the subject during the measurement.

Similarly, in the lateral direction, the displacement 240 may be displaced from the midline of where the structure 210 lies. Additionally, the displacement 240 in the lateral direction may be based on the coregistration between the images generated by the image system 124 and the OCT system 122 and the images may be aligned or matched from different frequencies to a common reference system. This coregistration ensures that images taken are at different times, from different angles, or using different imaging modalities may be aligned such that structure 210 is matched across all images taken.

Based on the displacement 240, feedback may be provided to the user via the display 140. The feedback may include, but is not limited to, that the displacement 240 has exceeded a preset threshold, the quality of the measurement has not met a predetermined threshold, the measurement at the location 220 on the structure 210 is displaced beyond a predetermined threshold compared to the measured location 230, a notification that the measurement has been discarded, and/or that a warning regarding the measurement. For example, feedback may also be provided based on coregistration of either the OCT system 122 or the image system 124 from previous measurements of the patient to new or current measurements of the patient. This alignment of coregistration of measurements may provide feedback to the user indicating that the coregistration has changed.

FIG. 3 illustrates a GUI 300 displaying a conductive hearing loss assessment, according to some embodiments. The GUI 300 may include a first image 354 in a first window 352, a second image 364 in a second window 362, a third image 374 in a third window 372, and an info display 384 in a fourth window 382, or any subset of these. The GUI 300 may be displayed on the display 140.

In some embodiments, a first image 354 may be an OCT image generated by the OCT system 122. The first image 354 may be displayed in the first window 352. On the first image 354, a crosshair line 310 may overlay the OCT image, which may indicate the location of Doppler lines. The Doppler lines may indicate a region with the vibration displacement amplitude information. The Doppler lines may also indicate the depth selected for imaging (not pictured).

A second image 364 may be a real-time image generated by the image system 124. The second image 364 may be displayed in the second window 362. A third image 374 may be a plot generated via the processor based on the measurement by the OCT system 122. The third image 374 may be displayed in the third window 372. The third image 374 may be generated once measurements of the ear 130 at various frequencies are completed by the OCT system 122. The third image 374 may include a plot of a plurality of mobility measurements at a plurality of frequencies via a plot.

The data of the plurality of mobility measurements may include error bars, which indicate the accuracy level of the measurement, and may be calculated via the processor 110. Confidence bands and other tools/measures used in statistical analysis to derive or represent information on uncertainties and estimations of data may be indicated in the plot. In addition, the plurality of mobility measurement may include the mobility of a typical hearing ear in comparison to the current ear 130 being measured. A typical hearing ear may be in the range, as indicated by region 320. By comparing the mobility measurement of a typical hearing ear versus the mobility measurement of the ear 130 being measured, the user may determine whether or not patient's hearing may have issues. The issues of the patient's hearing are visualized through the GUI 300 and through the GUI 300, feedback may be provided to the user. This allows for a user to address underlying ear health issues with data that may indicate hearing loss, fluid in the ear, or other hearing issues.

In addition, or alternatively, the plurality of mobility measurement may include the mobility of one or more past measurements of the same ear in comparison to the current ear 130 being measured. Past measurements of the same ear may be in the range, as indicated by region 320 or depicted as individual lines: Preferably past measurements are distinguishable from one another and from the current ear measurements, for example by color and/or style. In particular, it is preferred that the line color and/or style can indicate the timing of the measurements, for example in form of a time series. By comparing the mobility measurement of the hearing ear in the past versus the mobility measurement of the ear 130 being measured, the user may determine whether or not patient's hearing may have issues or has improved. The issues of the patient's hearing are visualized through the GUI 300. The issues of the patient's hearing are visualized through the GUI 300 and through the GUI, feedback may be provided to the user to assist with future measurements.

A typical, or past, hearing ear versus the ear 130 of the patient may vary based on a variety of factors such as age of the patient, sex of the patient, overall health of the patient, genetics, or the like. The range as indicated in region 320 may vary based on the factors described herein. A past hearing ear versus the current ear 130 of the patient may vary based on a variety of factors such as the source of the past data, the age of the past data, the trustworthiness of the past data and/or the error estimations of the past data or the like. The range as indicated in region 320 may vary based on the factors described herein. Additionally, the mobility measurement displayed in the fourth window 382 may vary based on which structure 210 of the ear 130 is being measured.

In some embodiments, within the fourth window 382, information regarding the measurement may be displayed in the info display 384. The information may include, but is not limited to, which ear 130 is being measured, which structure within the ear 130 is being measured, which measurement number is being taken a progress meter, a measurement at which frequency, a control panel, etc. For example, the user may use the control panel, indicated as foot pedals in window 384, to take the next set of measurements. Additionally, a foot panel (not pictured) may be connected to the processor 110 and the user may manually press the foot pedal to advance to the next measurement. Within window 374, the plurality of frequencies measured may be indicated by data points 330. For example, measurements were taken at 500 Hz, 700 Hz, 1000 Hz, 1350 Hz, 2000 Hz, and 3000 Hz and are indicated by measurements represented by data points 330,332, 334, and 336.

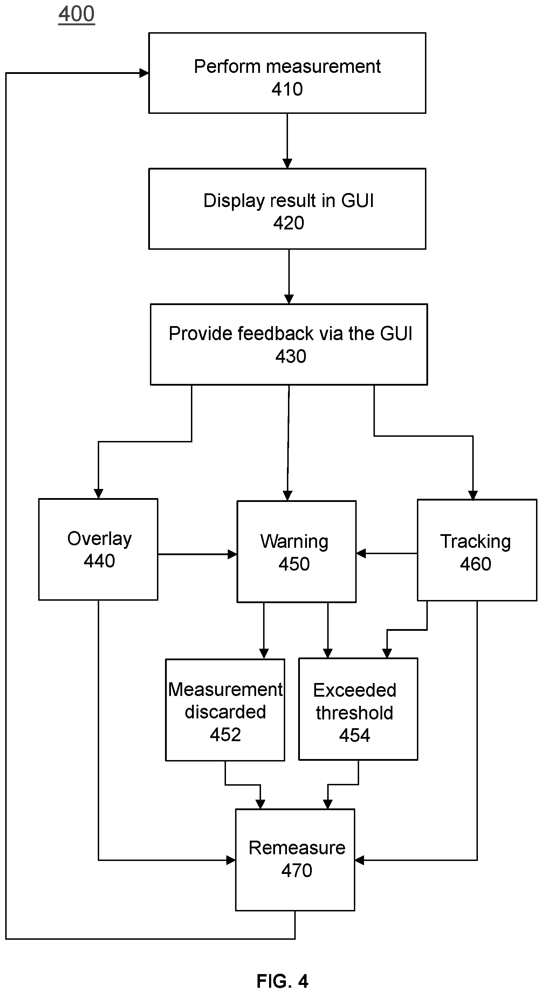

Past measurements of the same ear are exemplarily and schematically shown as data points and lines in fourth image 374. Data points correspond to red measurement 330, green measurement 332, orange measurement 334, and blue measurement 336. The color may indicate a time ordering, for example the red measurement 330 may correspond to the oldest measurement, the green measurement 332 to the second oldest measurement, the blue measurement 336 to the third oldest measurement before the current measurement, which is represented by the orange measurement 334. Instead of data points and lines, these measurements may also be shown with error bars or confidence bands and the like. Alternatively, or additionally, one or more of the data points and lines 330 to 336 may also correspond to the measurements of the same patient, but the other ear not currently measured. Alternatively, or additionally to color, any other property may be used to support the distinguishability of different measurements, for example shading, style, thickness and/or opacity. Similarly, the data points related to measurements 330 to 336 need not to be measured at the same frequencies as the data points related to orange measurement 334. Feedback may be provided to the user based on measurements 330 to 336, further described in FIG. 4.

Additionally, a foot panel, a voice control unit, a gesture control unit and/or a gaze control unit (all not pictured) may be connected to the processor 110 and the user may manually press the foot pedal or input a respective command according to the respective control unit to advance to the next measurement. Alternatively, or additionally, a respective input option, such as a button, may be provided on the vibrometry diagnostic system 120 and connected to the processor 110 and the user may manually execute the input option, for example press the button, to advance to the next measurement.

While images 354 364 374 correspond to systems within the vibrometry diagnostic system 120, the images may be displayed in any window 352 362 372 of the GUI 300. While windows 352 362 372 382 are displayed in a certain configuration within GUI 300, other configurations of the layout may be envisioned and the order of the windows 352 362 372 382 may be changed. For example, the first window 352 may be on the right side of the GUI 300 alternatively.

FIG. 4 illustrates a flowchart 400 for providing feedback to a user making measurements using an OCT system 122, according to some embodiments. The flowchart 400 shall be described with reference to FIGS. 1-3. However, the flowchart 400 is not limited to that example embodiment and may include other embodiments described herein.

Flowchart 400 illustrates the different types of feedback that may be provided to the user via the display 140. The feedback may be generated in the GUI 300 in the form of a pop-up window, overlays, an alarm, an alert, a notification, or the like. The feedback may allow the user to improve stability of the vibrometry diagnostic system 120, provide guidance to the user regarding the measured location 230, modify the position or orientation of the vibrometry diagnostic system 120, such as retracting or shifting the vibrometry diagnostic system 120 in any direction, misplacement of the vibrometry diagnostic system 120 in the ear 130, or the like.

In 410, the user may perform a measurement. The measurement may be on a structure 210 within the ear 130 of a patient using the vibrometry diagnostic system 120.

Once the measurement is completed, the processor 110 may perform various calculations or processing of data generating a result. In 420, the result may be displayed in the GUI 300 on the display 140. The results may include a plot, an image, data about the measurement, or the like. Based on the result generated, feedback may be generated. In 430, feedback may be provided to the user in the GUI 300. The feedback may include a form of communication that includes, but is not limited to, an overlay 440, a warning, 450, tracking 460, or the like.

For example, in the first image 354, the second image 364, or the third image 364, an overlay 440 in any of the images may be generated. The overlay 440 may be in the form of lines, a tracer, or a specific shape as a circle indicating a predetermined threshold. Additionally, the predefined threshold may be based on an intensity of reflected light generated from the OCT system 122 from the structure 210. For example, typical computer vision algorithms may identify the structure 210 being measured. The overlay 440 may indicate the boundary of the structure 210 and tracking 460, described herein, may indicate that the measured location 220 is no longer being measured on the structure 210.

In addition to computer vision algorithms, deep learning methods may be employed to provide feedback to the user via the GUI 300. For example, the user may input which structure 210 will be measured. Based on the input, deep learning methods may interpret the input, use previous medical history or previous measurements on the patient and provide feedback to the user in the form of predicted future measurements. The displacement 240 may be determined using image analysis or deep learning methods of the shape/contour of the depicted structure in the first image 354, the second image 364, or the third image 374.

The first image 354, the second image 364, or the third image 374 may be 2D, 3D, or 4D. With a more dimensional image, the user may receive feedback based direct visualization of the structure 210. For example, a 3D image may allow the user to position the vibrometry diagnostic system 120 in the right position based on the direct visualization. This representation of the structure 210 may assist the user in correlating delicate or slight movements of the vibrometry diagnostic system 120 versus magnification of the structure 210. Feedback may also be provided by comparing the first image 354, the second image 364, or the third image 374 of a structure 210 versus a typical image of the structure 210.

Feedback may be information regarding the displacement 240. The feedback may include, but is not limited to the position of the measurement being wrong; updated visualization, in real-time, in the form of a graphic or image; the direction of the displacement 240 with orientation in 3D; distance of the displacement 240; that the displacement 240 may exceed a predetermined threshold; or recommendation to the user how to minimize or reduce the displacement 240.

The warning 450 may indicate that the measurement may be discarded 452 or that the measurement has exceeded a predefined threshold 454. The warning 450 may be a sound alert, an alert, a notification, a pop-up window, or the like. The warning 450 may warn the user that there is a problem with the measurement or that its quality was insufficient. For example, the measurement may be discarded 452 when the displacement of the measured location 230 versus the location 220 sufficiently exceeds a predetermined threshold such that the measurement is no longer valid or useful. For example, the user may indicate that the location 220 of the measurement is at the umbo. However, if the measured location 230 is instead at the incus, the processor 110 may recognize the discrepancy as the displacement 240 is sufficiently large and the measurement may be discarded 452. Additionally, the warning 450 may alert the user if the displacement 240 is in the axial or lateral direction. While this provides one example of the measurement of the incus and umbo, other embodiments may be envisioned, other structures measured, and carried out through methods and systems described herein.

For example, the tracking 460 may be the form of feedback provided. The tracking 460 may be provided in the first image 354, the second image 364, or the third image 364, in the GUI 300. For example, a dot, of any color, may be provided on any of the first image 354, the second image 364, or the third image 364. The dot may indicate the location 220 of the region of interest and may be fixed. A circle, of a different color than the dot, may be tracking 460 the dot. If the dot is no longer in the circle, the measurement has left the region of interest. The tracking 460 may indicate that the dot is no longer in the circle in an axial or lateral direction along the structure 210. A warning 450 may be provided for such tracking 460 described herein. While this provides one example of the measurement using a dot and circle, other embodiments may be envisioned and carried out through methods and systems described herein.

In some embodiments, the tracking 460 may include specific circles that are colored to indicate that the displacement 240 is the axial or lateral direction. For example, in the axial direction, the circle may be green colored or in the lateral direction, the circle may be purple colored. Any colored circle may be envisioned for such tracking 460 on any of the first image 354, the second image 364, or the third image 364. Colors may also indicate whether or not the measurement, e. g. an image quality, is good or bad. For example, red may indicate that the measurement was insufficient and needs to be redone and green may indicate that a quality of the measurement was sufficient.

Based on either the overlay 440, the warning 450, or tracking 460, this may require new measurements. In 470, a remeasurement may be required. The flowchart 400 may start from the beginning with 410, the user performing a new measurement. By performing a new measurement, this may be considered updated feedback as the flowchart 400 would be repeated and then new measurements would be performed and updated feedback provided.

FIG. 5 illustrates a GUI 500 displaying feedback to a user, according to some embodiments. The GUI 500 may include a first image 354 in a first window 352, a second image 364 in a second window 362, a third image 374 in a third window 372, and an info display 384 in a fourth window 382. The GUI 300 may be displayed on the display 140. The GUI 500 of FIG. 5 may be as the GUI 300 described in FIG. 3, but includes how feedback may be displayed to a user, according to embodiments described herein.

The feedback that may be provided to the user may include but is not limited to, an overlay 440, a warning 450, and tracking 460, further described in FIG. 4. In some embodiments, the first window 352 may include a first image 354 that is an OCT image. Based on where the user is measuring within the ear 130, a left overlay 552 and a right overlay 554 may indicate boundaries as to where the measuring should be within. The GUI 500 may indicate to the user that the measurement has gone out of the boundaries indicated by the left overlay 552 and right overlay 554. While the left overlay 552 and right overlay 554 are depicted in the first image 354, the second image 364 may have similar overlay 440.

In some embodiments, the warning 450 may be displayed in the fourth window 382 or a popup in the GUI 500. The warning may indicate a predetermined threshold has been exceeded or that the measurement is out of a designated boundary. Additionally, tracking 460 may be depicted the second image 364 of the second window 362. A dot 562 may indicate the current location of the measurement. The region of interest may be indicated by circle 564. Once the user takes a measurement that is out of the circle 564, the user may be notified that the measurement is out of bounds. The notification may be in the form of a warning 450. While tracking 460 is depicted in the second image 364, the first image 354 may have similar tracking 460.

In some embodiments, coregistration of the OCT system 122 and image system 124 may be determined via the GUI 500. The first image 354 may be an OCT image including B-mode. Line 556 in the middle of the first image 354 indicates the location of where the measurement should ideally stay fixed. As described herein, small movements may cause a measurement to be shifted. This image may be coregistered with the real-time image generated by the image system 124 and depicted in the second image 364. The second image 364 may include line 566 to which line 556 is coregistered to. For example, the displacement 240 may be based on the coregistration between the images generated by the OCT system 122 and the image system 124 and the images may be aligned or matched from different frequencies to a common reference system via line 556 and line 566. This coregistration between the OCT system 122 and the image system 124 ensures that images taken at different times, from different angles, or using different imaging modalities may be aligned such that structure 210 is matched across all images taken.

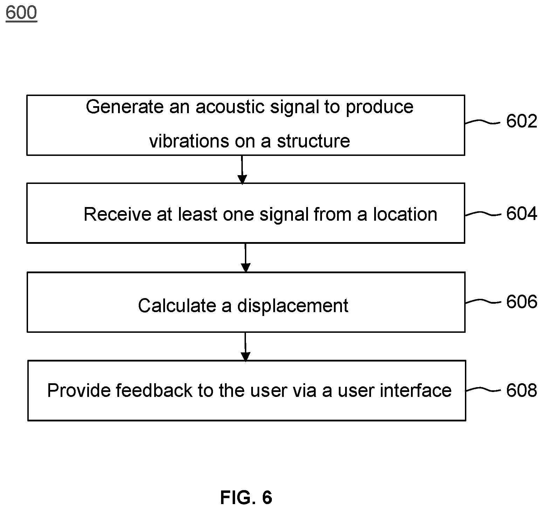

FIG. 6 illustrates a method 600 for providing feedback to a user via a vibrometry diagnostic system 120, according to some embodiments. Method 600 may be performed by processing logic that may include hardware (e.g., circuitry, dedicated logic, programmable logic, microcode, etc.), software (e.g., instructions executing on a processing device), or a combination thereof. It is to be appreciated that not all steps may be needed to perform the disclosure provided herein. Further, some of the steps may be performed simultaneously, or in a different order than shown in FIG. 6, as will be understood by a person of ordinary skill in the art.

Method 600 shall be described with reference to FIGS. 1-5. However, method 600 is not limited to that example embodiment.

In step 602, an acoustic stimulus may be generated to produce vibrations on a structure. The structure may be an ossicle of a middle ear, an incus of an ear, or an umbo of the ear. For example, the acoustic system 126 generates the acoustic stimulus to produce vibrations on a structure 210 on the ear 130. The structure 210 may be an ossicle of a middle ear, an incus of the ear 130, or an umbo of the ear 130.

In step 604, at least one signal may be received from a location. The at least one signal may include vibrometry measurement of a vibration of the structure at the location. For example, the OCT system 122 may receive at least one signal from the location 220 on the structure 210 of the vibration induced from the acoustic system 126 at the ear 130.

In step 606, a displacement may be calculated. The displacement may be a distance by which a measurement location is displaced from the location. The displacement is in either the axial direction or the lateral direction along the structure. The displacement may be based on an image of a target region containing the structure. The displacement is based on motion during measuring the at least one signal. For example, a displacement 240 may be calculated that includes a distance from a measured location 230 is displaced from the location 220. The displacement 240 may be in the axial direction or the lateral direction along the structure 210. Further, the displacement 240 may be based on the first image 354, second image 364, and third image 374 of

In step 608, feedback may be provided to the user via a user interface. The feedback may include visualization of the displacement. The method 600 may include providing a warning to the user when the displacement exceeds a predefined threshold. The method 600 may further include generating an image of the structure and providing the feedback as an overlay in the image. For example, feedback may be provided to the user via a display 140. The feedback may include visualization, such as a plot or an image, of the displacement 240. An image, such as first image 354, second image 364, or third image 374 may be generated and displayed. The feedback may include an overlay on any one of the first image 354, second image 364, or third image 374. The method 600 may include providing a warning to the user when the displacement 240 exceeds a predefined threshold, which may be based on an intensity of reflected light from the structure 210 due to the OCT system 122. Based on the warning, updated feedback may be provided to the user via the display 140.

Various embodiments may be implemented, for example, using one or more well-known computer systems, such as computer system 700 shown in FIG. 7. For example, the vibrometry diagnostic system 120 may be implemented using combinations or sub-combinations of computer system 700. Also, or alternatively, one or more computer systems 700 may be used, for example, to implement any of the embodiments discussed herein, as well as combinations and sub-combinations thereof.

FIG. 7 shows a computing device 700 for implementing various embodiments, according to some embodiments. For example, computing device 700 may function as system of the vibrometry diagnostic system 120 or any portion(s) thereof, or multiple computing devices 600 may function as a system of the vibrometry diagnostic system 120.

Computing device 700 may be implemented on any electronic device that runs software applications derived from compiled instructions, including without limitation personal computers, servers, smart phones, media players, electronic tablets, game consoles, email devices, etc. In some implementations, computing device 700 may include one or more processors 110, one or more input devices 704, one or more display devices 706, one or more communication interfaces 708, and one or more computer-readable medium 710. Each of these components may be coupled by bus 718, and in some embodiments, these components may be distributed among multiple physical locations and coupled by a network.

Control unit 740 may assist in providing instructions to the vibrometry diagnostic system 120. The control unit 740 may send an electronic control signal to the vibrometry diagnostic system 120. In response to receiving the control signal, the vibrometry diagnostic system 120 may perform measurements. The surgeon or another user may design a configuration of the control unit 740 by creating the instructions and providing the instructions to the computer device 700. For example, the instructions may be uploaded to a database of the computer device 700 or the surgeon may use input device 704 to perform measurements using the vibrometry diagnostic system 120.

Display device 706 may be any known display technology, including but not limited to display devices using Liquid Crystal Display (LCD) or Light Emitting Diode (LED) technology. Processor(s) 110 may use any known processor technology, including but not limited to graphics processors and multi-core processors. Input device 704 may be any known input device technology, including but not limited to a keyboard (including a virtual keyboard), mouse, controller, joystick, track ball, and touch-sensitive pad or display.

Bus 718 may be any known internal or external bus technology, including but not limited to ISA, EISA, PCI, PCI Express, NuBus, USB, Serial ATA or FireWire. In some embodiments, some or all devices shown as coupled by bus 718 may not be coupled to one another by a physical bus, but by a network connection, for example. Computer-readable medium 710 may be any medium that participates in providing instructions to processor(s) 110 for execution, including without limitation, non-volatile storage media (e.g., optical disks, magnetic disks, flash drives, etc.), or volatile media (e.g., SDRAM, ROM, etc.).

Computer-readable medium 710 may include various instructions for implementing an operating system 712 (e.g., Mac OS, Windows, Linux). The operating system 712 may be multi-user, multiprocessing, multitasking, multithreading, real-time, and the like. The operating system 712 may perform basic tasks, including but not limited to: recognizing input from input device 704; sending output to display device 706; keeping track of files and directories on computer-readable medium 710; controlling peripheral devices (e.g., disk drives, printers, etc.) which may be controlled directly or through an I/O controller; and managing traffic on bus 718. Network communication 714 may establish and maintain network connections (e.g., software for implementing communication protocols, such as TCP/IP, HTTP, Ethernet, telephony, etc.).

Application(s) and program module(s) 716 may be an application that uses or implements the outcome of processes described herein and/or other processes. For example, application(s) 716 may provide UI and/or UI elements for displaying and/or manipulating updates identified by computer device 700 as described above. In some embodiments, the various processes may also be implemented in operating system 712.

The described features may be implemented in one or more computer programs that may be executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., Objective-C, Java), including compiled or interpreted languages, and it may be deployed in any form, including as a stand-alone program or as a module, component, subroutine, or other unit suitable for use in a computing environment.

Suitable processors 110 for the execution of a program of instructions may include, by way of example, both general and special purpose microprocessors, and the sole processor or one of multiple processors or cores, of any kind of computer. Generally, a processor 110 may receive instructions and data from a read-only memory or a random-access memory or both. The essential elements of a computer may include a processor 110 for executing instructions and one or more memories for storing instructions and data. Generally, a computer may also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data may include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

To provide for interaction with a user, the features may be implemented on a computer having a display device such as an LED or LCD monitor for displaying information to the user and a keyboard and a pointing device such as a mouse or a trackball by which the user may provide input to the computer.

The features may be implemented in a computer system that includes a back-end component, such as a data server, or that includes a middleware component, such as an application server or an Internet server, or that includes a front-end component, such as a client computer having a graphical user interface or an Internet browser, or any combination thereof. The components of the system may be connected by any form or medium of digital data communication such as a communication network. Examples of communication network include, e.g., a telephone network, a LAN, a WAN, and the computers and networks forming the Internet.

The computer system may include clients and servers. A client and server may generally be remote from each other and may typically interact through a network. The relationship of client and server may arise by virtue of computer programs running on the respective computers and having a client-server relationship to each other.

One or more features or steps of the disclosed embodiments may be implemented using an API and/or SDK, in addition to those functions specifically described above as being implemented using an API and/or SDK. An API may define one or more parameters that are passed between a calling application and other software code (e.g., an operating system, library routine, function) that provides a service, which provides data, or that performs an operation or a computation. SDKs may include APIs (or multiple APIs), integrated development environments (IDEs), documentation, libraries, code samples, and other utilities.

The API and/or SDK may be implemented as one or more calls in program code that send or receive one or more parameters through a parameter list or other structure based on a call convention defined in an API and/or SDK specification document. A parameter may be a constant, a key, a data structure, an object, an object class, a variable, a data type, a pointer, an array, a list, or another call. API and/or SDK calls and parameters may be implemented in any programming language. The programming language may define the vocabulary and calling convention that a programmer will employ to access functions supporting the API and/or SDK.

In some implementations, an API and/or SDK call may report to an application the capabilities of a device running the application, such as input capability, output capability, processing capability, power capability, communications capability, etc.

While this disclosure describes exemplary embodiments for exemplary fields and applications, it should be understood that the disclosure is not limited thereto. Other embodiments and modifications thereto are possible and are within the scope and spirit of this disclosure. For example, and without limiting the generality of this paragraph, embodiments are not limited to the software, hardware, firmware, and/or entities illustrated in the figures and/or described herein. Further, embodiments (whether or not explicitly described herein) have significant utility to fields and applications beyond the examples described herein.

Embodiments have been described herein with the aid of functional building blocks illustrating the implementation of specified functions and relationships thereof. The boundaries of these functional building blocks have been arbitrarily defined herein for the convenience of the description. Alternate boundaries can be defined as long as the specified functions and relationships (or equivalents thereof) are appropriately performed. Also, alternative embodiments can perform functional blocks, steps, operations, methods, etc. using orderings different than those described herein.

References herein to "one embodiment," "an embodiment," "an example embodiment," or similar phrases, indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of persons skilled in the relevant art(s) to incorporate such feature, structure, or characteristic into other embodiments whether or not explicitly mentioned or described herein. Additionally, some embodiments can be described using the expression "coupled" and "connected" along with their derivatives. These terms are not necessarily intended as synonyms for each other. For example, some embodiments can be described using the terms "connected" and/or "coupled" to indicate that two or more elements are in direct physical or electrical contact with each other. The term "coupled," however, can also mean that two or more elements are not in direct contact with each other, but yet still co-operate or interact with each other.

The breadth and scope of this disclosure should not be limited by any of the above-described exemplary embodiments, but should be defined only in accordance with the following claims and their equivalents.

## Claims

1. A computer-implemented method for providing feedback to a user via an OCT (Optical Coherence Tomography) imaging system (120, 122) comprising:
generating a mechanical stimulus to induce vibrations (602) on an anatomical structure (210) in a body (130);
receiving OCT signal data (354) from a measured location (230) in the body (130, 604), wherein the OCT signal data comprises a vibrometry measurement of a vibration of the anatomical structure (210) at the measured location (230);
determining a displacement (240) from the OCT signal data (354), wherein the displacement (240) is a spatial deviation by which the measured location (230) in the body (130) is displaced from a target location (220) in the body; and
providing feedback to the user via a user interface (140, 382, 384, 430, 608), wherein the feedback comprises visualization (140, 440) of the displacement (240).

2. The method of claim 1, further comprising:
generating a real-time image (364) by an image system (124) of the anatomical structure (210); and
providing the feedback (430) as an overlay (440) on the real-time image (364).

3. The method of claims 1 or 2, wherein the OCT signal data (354) comprises motion artifacts.

4. The method of claim 2 or 3wherein a first motion causing the motion artifacts has a lower frequency than a second motion of the vibration of the anatomical structure (210).

5. The method of one of claims 1 to 4, wherein the motion artifacts relate to a movement of the body (130) relative to an optical path of the OCT imaging system (120).

6. The method of one of claims 1 to 5, wherein the target location (220) comprises at least a portion of the anatomical structure (210) and the measured location (230) is different from the anatomical structure (210).

7. The method of one of claims 1 to 6, wherein an axial direction and a lateral direction of the displacement (240) relative to a direction of an optical path of the OCT imaging system (120, 122) are determined independently of each other.

8. The method of one of claims 1 to 8, wherein a lateral direction of the displacement (240) relative to a direction of an optical path of the OCT imaging system (120, 122) is based on coregistration of the OCT signal data (354) and the real-time image.

9. The method of one of claims 1 to 8, wherein the feedback to the user (430) comprises a recommendation on how to reduce or avoid the displacement, preferably based on an analysis of root causes of the displacement (240).

10. The method of one of claims 1 to 9, wherein the displacement (240) is communicated (450) to the user if it exceeds a predefined threshold (454).

11. The method of one of claims 10, wherein the predefined threshold is based on an intensity distribution of reflected light from the anatomical structure.

12. The method of one of claims 1 to 11, wherein the anatomical structure (210) is an ossicle of a middle ear (130).

13. The method of one of claims 1 to 12, wherein the displacement (240) is determined based on the real-time image of a target region (364) of the body (130) behind which the target region (364) the anatomical structure (210) is located along an optical path of the OCT imaging system (120, 122).

14. A computer program product for providing feedback to a user via an OCT (Optical Coherence Tomography) imaging system (120, 122), the computer program product comprising: a computer-readable storage medium (710) having program instructions provided thereon, the program instructions being executable by one or more computer systems or controllers to cause the one or more computer systems to perform the method of one of claims 1 to 13.

15. A device for providing feedback to a user via an OCT (Optical Coherence Tomography) imaging system (120, 122) comprising:
a mechanical stimulus source (126) configured to generate a mechanical stimulus to induce vibrations on an anatomical structure (210) in a body (130);
an OCT imaging device (122) configured to measure OCT signal data (354) from a measured location (230) in the body (130), wherein the OCT signal data (354) comprise a vibrometry measurement of a vibration of the anatomical structure (210) at the measured location (230);
a processor (110) and a memory connected to the mechanical stimulus source (126) and the OCT imaging system (120), wherein the processor (110) is configured to execute instructions stored in said memory for performing the steps of:
determining a displacement (240, 606) from the OCT signal data, wherein the displacement (240) is a spatial deviation by which the measured location (230) in the body (130) is displaced from a target location (220) in the body (130); and
providing feedback to the user via a user interface (140, 382, 384, 430, 608), wherein the feedback comprises visualization (140, 440) of the displacement (240).
